# EUROPEAN PATENT SPECIFICATION

(11) **EP 1 321 144 B1**
(45) Date of publication and mention of the grant of the patent: **08.12.2010**
(21) Application number: 01965597.6
(22) Date of filing: 13.09.2001
(51) Int. Cl.: A61K 9/08, A61K 31/5575, A61K 47/34, A61K 47/44, A61K 47/18, A61K 47/10, A61P 27/02

(54) **EYE DROPS**
AUGENTROPFEN
COLLYRE

(30) Priority: 13.09.2000 JP 2000277554
(43) Date of publication of application: 25.06.2003
(73) Proprietor: SANTEN PHARMACEUTICAL CO., LTD., Higashiyodogawa-ku, Osaka-shi, Osaka 533-8651 (JP); ASAHI GLASS COMPANY LTD., Tokyo 100-8405 (JP)
(72) Inventor: MORISHIMA, Kenji, c/o SANTEN PHARMACEUT. CO., LTD, Osaka-shi, Osaka 533-8651 (JP); KIMURA, Akio, c/o SANTEN PHARMACEUT. CO., LTD, Osaka-shi, Osaka 533-8651 (JP); ASADA, Hiroyuki, c/o SANTEN PHARMACEUT. CO., LTD, Osaka-shi, Osaka 533-8651 (JP); UMEDA, Masayuki, c/o SANTEN PHARMACEUT. CO., LTD, Osaka-shi, Osaka 533-8651 (JP); KUWANO, Mitsuaki, c/o SANTEN PHARMACEUT. CO., LTD, Osaka-shi, Osaka 533-8651 (JP)
(74) Representative: Peaucelle, Chantal
(86) International application number: PCT/JP2001/007928
(87) International publication number: WO 2002/022131

(56) References cited:
- EP-A- 0 603 800
- EP-A1- 0 603 800
- EP-A1- 0 930 296
- EP-A2- 0 850 926
- WO-A-00/03736
- US-A- 5 486 540
- US-A- 5 631 287

## Description

### Technical Field

The present invention relates to ophthalmic solutions comprising prostaglandin derivatives which are liable to be adsorbed to a container made of resin and hardly soluble in water as active ingredients, **characterized in that** concentrations of the prostaglandin derivatives in the ophthalmic solutions are prevented from lowering by adding a nonionic surfactant and/or an antioxidant.

### Background Art

Natural prostaglandins are well-known as substances having various physiological activities. Using these prostaglandins as leading compounds, many prostaglandin derivatives have been researched. For example, as prostaglandin derivatives to be used for ophthalmic use, it is known that prostaglandin derivatives disclosed in Published Japanese Translation of PCT No. 501025/1991, and Japanese Laid-open Patent Publication Nos. 108/1990 and 71344/1999 are useful as therapeutic agents for glaucoma or ocular hypertension having intraocular pressure lowering effects.

As mentioned above, the prostaglandin derivatives are useful as the therapeutic agents for glaucoma or ocular hypertension, but some prostaglandin derivatives are hardly soluble in water and liable to be adsorbed to a resinous container. In order to formulate these prostaglandin derivatives in ophthalmic solutions, it is necessary to solve the problem of the solubility in water and a problem of a lowering in drug concentration due to the adsorption to the container. Since some prostaglandin derivatives are liable to decompose when dissolved in water, it is necessary to solve the problem of stability in order to formulate these prostaglandin derivatives in ophthalmic solutions. Since the adsorption of the drug to eye droppers and the decomposition of the drug in the ophthalmic solutions lead to a lowering in drug concentration in the ophthalmic solutions, it is an important subject for preparing ophthalmic solutions to solve these problems.

### Disclosure of the Invention

Accordingly, the present inventors studied precisely a process for formulating prostaglandin derivatives according to claim 1 which are liable to be adsorbed to a container made of resin and hardly soluble in water, into ophthalmic solutions. As a result, it was found that solubility of the prostaglandin derivatives in water is increased and adsorption thereof to the resinous container can be remarkably inhibited by adding a nonionic surfactant such as polysorbate 80 to the ophthalmic solutions. It was also found that decomposition of the prostaglandin derivatives can be remarkably inhibited by adding an antioxidant such as disodium ethylenediaminetetraacetate

The present invention relates to the ophthalmic solutions comprising the prostaglandin derivatives according to claim 1 which are liable to be adsorbed to the container made of resin and hardly soluble in water (hereinafter referred to as "the prostaglandin derivatives") as active ingredients, **characterized in that** concentrations of the prostaglandin derivatives in the ophthalmic solutions are prevented from dropping by adding the nonionic surfactant and/or the antioxidant, and a method of preventing the concentrations from lowering.

The prostaglandin derivatives according to claim 1 are liable to be adsorbed to the resinous container and hardly soluble in water. Prostaglandin derivatives are prostaglandin F2α derivatives having fluorine atoms in their molecules disclosed in Japanese Laid-open Patent Publication Nos. 71344/1999 and 251225/1998. Prostaglandin derivatives are difluoroprostaglandin F2α derivatives disclosed in Japanese Laid-open Patent Publication No. 71344/1999. Prostaglandin derivatives are difluoroprostaglandin F2α derivatives having two fluorine atoms at the 15th-position disclosed in Japanese Laid-open Patent Publication No. 71344/1999. The prostaglandin derivatives are 16-phenoxy-15-deoxy-15,15-difluoro-17,18,19,20-tetranorprostaglandin F2α, 16-(3-chlorophenoxy)-15-deoxy-15,15-difluoro-17,18,19,20-tetranorprostaglandin F2α, 16-phenoxy-15-deoxy-15,15-difluoro-13,14-dihydro-17,18,19,20 tetranorprostaglandin F2α, alkyl esters thereof and salts thereof. Specific examples of the alkyl esters are lower alkyl esters such as methyl esters, ethyl esters, propyl esters, isopropyl esters, tert-butyl esters, pentyl esters and hexyl esters.

In the ophthalmic solution of the present invention, the prostaglandin derivatives are in a state where they are dissolved in water.

The expression "the prostaglandin derivatives are liable to be adsorbed to the resinous container" means that when the prostaglandin derivatives are stored in the resinous container in the form of an aqueous solution, a remaining rate (the remaining rate is a ratio of an amount of a prostaglandin derivative which keeps being effectively dissolved in the ophthalmic solution to an amount of a prostaglandin derivative which was dissolved) drops remarkably. For example, when a concentration of a prostaglandin derivative in an aqueous solution is 0.001% (The "%" means % by weight as far as there is no proviso. The same definition is applied hereinafter.), the above-mentioned expression means a state where 40% or more (remaining rate in the solution: less than 60%), usually 40 to 60%, typically about 50% of the compound is adsorbed to a container made of polyethylene or polypropylene after the compound was stored in the container at 40°C for six months.

The prostaglandin derivatives which are hardly soluble in water are derivatives which require 1,000 ml or more of water in order to dissolve 1 g of the derivatives (the 13th revised Japanese Pharmacopoeia explanatory, general rule A-51 (1996)).

Polysorbate 80 nonionic surfactants are added in order to prevent the concentration of the prostaglandin derivatives from lowering by improving water-solubility of the prostaglandin derivatives in the ophthalmic solution and by inhibiting the adsorption to the resinous container. Examples of nonionic surfactants are polyoxyethylene fatty esters such as polysorbate 60 [poly(oxyethylene)sorbitan monostearate], polysorbate 40 [poly(oxyethylene)sorbitan monopalmitate], poly(oxyethylene)sorbitan monolaurate, poly(oxyethylene)sorbitan trioleate and polysorbate 65 [poly(oxyethylene)sorbitan tristearate], polyoxyethylene hydrogenated castor oils such as polyoxyethylene hydrogenated castor oil 10, polyoxyethylene hydrogenated castor oil 40, polyoxyethylene hydrogenated castor oil 50 and polyoxyethylene hydrogenated castor oil 60, polyoxyethylene polyoxypropylene glycols such as polyoxyethylene (160) polyoxypropylene (30) glycol [Pluronic F68], polyoxyethylene (42) polyoxypropylene (67) glycol [Pluronic P123], polyoxyethylene (54) polyoxypropylene (39) glycol [Pluronic P85], polyoxyethylene (196) polyoxypropylene (67) glycol [Pluronic F127] and polyoxyethylene (20) polyoxypropylene (20) glycol [Pluronic L-44], polyoxyl 40 stearate and sucrose fatty esters. These nonionic surfactants can be used solely or in combination.

The nonionic surfactants, polysorbate 80, [poly(oxyethylene)sorbitan monooleate] and polyoxyethylene hydrogenated castor oil 60, are widely used as additives of ophthalmic solutions.

Ethylenediaminetetraacetic acid antioxidants are added in order to prevent the concentration of the prostaglandin derivatives from lowering by inhibiting decomposition of the prostaglandin derivatives in the ophthalmic solution. Examples of antioxidants are sodium nitrite, ascorbic acid, L-ascorbic acid stearate, sodium hydrogensulfite, alphathioglycerin, erythorbic acid, cysteine hydrochloride, citric acid, tocopherol acetate, potassium dichloroisocyanurate, dibutylhydroxytoluene, 2,6-di-t-butyl-4-methylphenol, soybean lecithin, sodium thioglycollate, sodium thiomalate, natural vitamin E, tocopherol, ascorbyl pasthyminate, sodium pyrosulfite, butylhydroxyanisole, 1,3-butylene glycol, pentaerythtyl tetrakis[3-(3,5-di-t-butyl-4-hydroxyphenyl)]propionate, propyl gallate, 2-mercaptobenzimidazole and oxyquinoline sulfate. The antioxidants can be used solely or in combination.

Ethylenediaminetetraacetic acid, salts thereof and dibutylhydroxytoluene, are widely used as additives of ophthalmic solutions. It is particularly preferable to combine ethylenediaminetetraacetic acid or the salt thereof with dibutylhydroxytoluene.

Examples of materials of the resinous container are polyethylene, polypropylene, polyethylene terephthalate, polyvinyl chloride, acrylic resins, polystyrene, polymethyl methacrylate and nylon 6. Preferred examples of the materials are polyethylene, polypropylene and polyethylene terephthalate. These resins can be high-density resins or low-density resins.

An amount (concentration) of the prostaglandin derivatives in the ophthalmic solution can be appropriately selected depending on object diseases, symptoms and the like, and is preferably 0.00005 to 0.05%.

An amount (concentration) of nonionic surfactants in the ophthalmic solution can be appropriately increased or decreased depending on the amount of the prostaglandin derivatives. It is preferable to select the concentration of nonionic surfactants which is five or more times that of the prostaglandin derivatives from the viewpoint of an increase in water-solubility of the prostaglandin derivatives. Further, it is preferable to select the concentration of nonionic surfactants which is ten or more times that of the prostaglandin derivatives from the viewpoint of a more certain assurance of water-solubility. The higher the concentration of the nonionic surfactants, the higher is the water-solubility of the prostaglandin derivatives. Accordingly, an upper limit of the concentration has no theoretical limitation, but is naturally required from the viewpoint of use for the ophthalmic solution. Namely, when nonionic surfactants are added at a high concentration, they exert adverse effects on ocular tissues such as cornea. Accordingly, the concentration of nonionic surfactants in the ophthalmic solution is usually 0.5% or less regardless of the concentration of the active ingredient.

An amount (concentration) of antioxidants in the ophthalmic solution can be appropriately selected depending on the kind of antioxidants. For example, when the antioxidant is disodium ethylenediaminetetraacetate, the concentration is usually 0.005 to 0.5%, preferably 0.01 to 0.1%. When the antioxidant is dibutylhydroxytoluene, the concentration is usually 0.00001 to 0.001%, preferably 0.00005 to 0.0005%.

Effects of the present invention are described in detail in later Examples. The water-solubility of the prostaglandin derivatives was improved and the adsorption thereof to the resinous container was remarkably inhibited by adding the nonionic surfactant polysorbate 80 to the ophthalmic solutions. The decomposition of the prostaglandin derivatives in the ophthalmic solutions was effectively inhibited by adding the antioxidant disodium ethylenediaminetetraacetate. These experimental results show that the concentration of the prostaglandin derivatives in the ophthalmic solution can be remarkably prevented from lowering.

When the ophthalmic solution of the present invention is prepared, pharmaceutically acceptable various additives such as an isotonic agent such as sodium chloride, potassium chloride, calcium chloride, glycerin or propylene glycol, a buffering agent such as boric acid, borax, citric acid, disodium hydrogen phosphate or s -aminocaproic acid, and a preservative such as benzalkonium chloride, chlorhexidine gluconate, benzethonium chloride, sorbic acid, potassium sorbate, ethyl p-hydroxybenzoate or butyl p-hydroxybenzoate can be added in addition to the above-mentioned nonionic surfactants and antioxidants.

pH of the ophthalmic solution of the prostaglandin derivatives is preferably 3 to 8, particularly 4 to 7.

The ophthalmic solution of the present invention can be prepared by a widely-used process without special technique and operation.

It is hereinafter shown by Examples that the ophthalmic solutions of the present invention effectively prevent the concentration of the prostaglandin derivatives from lowering. These Examples do not limit the scope of the present invention, but are intended to make the present invention more clearly understandable.

### Brief Description of Drawing

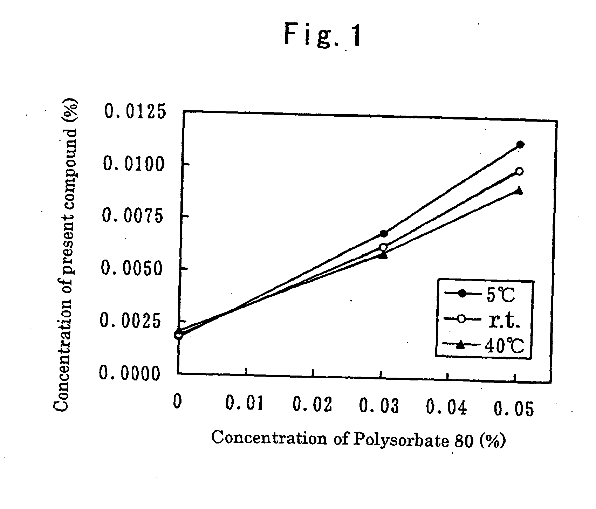

Fig. 1 is a graph showing effects of polysorbate 80 concentrations on solubility of the 16-phenoxy-15-deoxy-15,15-difluoro-17,18,19,20-tetranorprostaglandin F2α isopropyl ester.

### Best Mode for Carrying out the Invention

16-Phenoxy-15-deoxy-15,15-difluoro-17,18,19,20-tetranorprostaglandin F2α isopropyl ester (hereinafter referred to as "the present compound") was hereinafter used as a typical example of the prostaglandin derivatives in Examples.

### 1. Stability test 1

An effect of addition of a nonionic surfactant on preventing the present compound from adsorbing to a resinous container was studied. Remaining rates of the present compound were measured in a solution to which polysorbate 80 was added as the nonionic surfactant (formulation 1), a solution to which polyoxyethylene hydrogenated castor oil 60 (hereinafter referred to as "HCO60") was added as the nonionic surfactant (formulation 2) and a solution to which the nonionic surfactant was not added as control 1. Table 1 shows the concentrations of the components. The "%" in the table is % by weight.

**Table 1**

| Components | Control 1 | Comparative Formulation 1 | Comparative Formulation 2 |
|---|---|---|---|
| Present compound | 0.001% | 0.001% | 0.001% |
| Polysorbate 80 | | 0.01% | |
| HCO60 | | | 0.01% |

Test method: Each solution was filleld into a container made of polyethylene and a container made of polypropylene and stored at 40 °C for six months. Then each remaining rate of the present compound in the solution was measured by a high-performance liquid chromatography method (hereinafter referred to as "the HPLC method").
Results and consideration: Table 2 shows results measured by the HPLC method.

**Table 2**

| | Control 1 | Comparative Formulation 1 | Comparative Formulation 2 |
|---|---|---|---|
| Remaining rates of the present compound in polyethylene container | 42% | 72% | 63% |
| Remaining rates of the present compound in polypropylene container | 56% | 83% | 80% |

From Table 2, it is found that the remaining rate of the present compound in the solution of the formulation 1 or the formulation 2 to which the nonionic surfactant was added is higher than that of the control 1 to which the nonionic surfactant was not added in both resinous containers of polyethylene and polypropylene, and adsorption of the present compound to the resinous containers was remarkably inhibited.

### 2. Stability test 2

An inhibitory effect of addition of an antioxidant on decomposition of the present compound was studied. Remaining rates of the present compound were measured in a solution to which disodium ethylenediaminetetraacetate (hereinafter referred to as "the EDTA salt") was added as the antioxidant (formulation 3) and a solution to which the antioxidant was not added as control 2. Polysorbate 80 was added in an amount of 0.05% in each formulation as a solubilizing agent of the present compound. In order to evaluate only decomposition of the present compound, a glass container, which hardly exhibits adsorptivity of the present compound, was used as a storage container. Further, iron(III) chloride, which has concentrations described in terms of iron ion concentrations in Table 3, was added as a substance which promotes the decomposition of the present compound. The "%" in the table is % by weight.

**Table 3**

| Components | Control 2 | Formulation 3 |
|---|---|---|
| Present compound | 0.005% | 0.005% |
| EDTA salt | - | 0.05% |
| Iron ion | 0.01 ppm | 0.01 ppm |

Test method: Each solution was filled into the glass container and stored at 40°C for six months. Then the remaining rate of the present compound in the solution was measured by the HPLC method. Results and consideration: Table 4 shows results measured by the HPLC method.

**Table 4**

| | Control 2 | Formulation 3 |
|---|---|---|
| Remaining rate of the present compound | 22% | 79% |

From Table 4, it is found that the remaining rate of the present compound in the solution of the formulation 3 to which the EDTA salt was added is higher than that of the control 2 to which the EDTA salt was not added, and the decomposition of the present compound was remarkably inhibited.

### 3. Stability test 3

An inhibitory effect of combined use of two antioxidants on decomposition of the present compound was studied. Remaining rates of the present compound were measured in a solution to which the two antioxidants, i.e., the EDTA salt and dibutylhydroxytoluene were added as the antioxidants (formulation 4) and a solution to which the antioxidants were not added as control 3. Polysorbate 80 was added in an amount of 0.05% in each formulation as a solubilizing agent of the present compound. In order to evaluate only decomposition of the present compound, a glass container, which hardly exhibits adsorptivity of the present compound, was used as a storage container. Further, a storage temperature was raised to 60°C in order to promote the decomposition of the present compound. The "%" in the table is % by weight.

**Table 5**

| Components | Control 3 | Formulation 4 |
|---|---|---|
| Present compound | 0.005% | 0.005% |
| EDTA salt | - | 0.05% |
| Dibutylhydroxytoluene | - | 0.0001% |

Test method: Each solution was filled into the glass container and stored at 60°C for two weeks. Then the remaining rate of the present compound in the solution was measured by the HPLC method. Results and consideration: Table 6 shows results measured by the HPLC method.

**Table 6**

| | Control 3 | Formulation 4 |
|---|---|---|
| Remaining rate of the present compound | 19.3% | 99% |

From Table 6, it is found that the remaining rate of the present compound in the solution of the formulation 4 to which the EDTA salt and dibutylhydroxytoluene were added as the antioxidants is higher than that of the control 3 to which the antioxidants were not added, and the decomposition of the present compound was remarkably inhibited.

### 4. Stability test 4

Effects of addition of the nonionic surfactant and the antioxidant on preventing the present compound from adsorbing to the resinous container and on inhibiting decomposition of the present compound were studied. A remaining rate of the present compound was measured in an ophthalmic solution to which polysorbate 80 and the EDTA salt were added as the nonionic surfactant and the antioxidant respectively (formulation 5). The "%" in the table is % by weight.

**Table 7**

| Components | Formulation 5 |
|---|---|
| Present compound | 0.005% |
| Polysorbate 80 | 0.05% |
| EDTA salt | 0.05% |

Test method: The ophthalmic solution of formulation 5 was filled into a container made of polypropylene and stored at 40°C for six months, and then the remaining rate of the present compound in the ophthalmic solution was measured by the HPLC method.
Result and consideration: Table 8 shows a result measured by the HPLC method.

**Table 8**

| | Formulation 5 |
|---|---|
| Remaining rate of the present compound | 95.6% |

From Table 8, it is found that the remaining rate of the present compound is high even after the ophthalmic solution of formulation 5 was stored in the container made of polypropylene for a long period, and a concentration of the present compound in the ophthalmic solution was remarkably prevented from lowering.

### 5. Solubility test

In order to formulate a drug which is hardly soluble in water in an ophthalmic solution, it is necessary to devise to dissolve the drug in water. Since the nonionic surfactant acts as the solubilizing agent, the following solubility tests were performed in order to make sure of its required amount.
Test method: The present compound having a concentration exceeding solubility and polysorbate 80 were added to 10 ml of water, the mixtures were stirred at 5°C, room temperature and 40°C for 24 hours respectively and then centrifuged at 20,000 rpm, and concentrations of the present compound contained in the supernatants were measured by the HPLC method.
Results and consideration: Fig. 1 shows results measured by the HPLC method. The "%" in the figure is % by weight.

From Fig. 1, it is found that solubility of the present compound increases depending on amounts of polysorbate 80, and the amounts of polysorbate 80 (nonionic surfactant) are preferably five or more times the concentration of the present compound, considering storage conditions and a change in concentration of the present compound. Water-solubility of the present compound at low temperatures is higher than that at high temperatures.

Effects of the present invention are as follows. The solubility of the prostaglandin derivatives in water is improved and the adsorption thereof to the resinous container is remarkably inhibited by adding nonionic surfactants such as polysorbate 80 and polyoxyethylene hydrogenated castor oil 60 to the ophthalmic solutions. The decomposition of the prostaglandin derivatives in the ophthalmic solutions is effectively inhibited by adding antioxidants such as disodium ethylenediaminetetraacetate and dibutylhydroxytoluene. These experimental results show that the concentrations of the prostaglandin derivatives in the ophthalmic solution were remarkably prevented from lowering.

### Industrial Applicability

The present invention provides ophthalmic solutions comprising prostaglandin derivatives which are liable to be adsorbed to a resinous container and hardly soluble in water as active ingredients, **characterized in that** concentrations of the prostaglandin derivatives in the ophthalmic solutions are prevented from lowering by adding a nonionic surfactant and/or an antioxidant.

## Claims

1. An ophthalmic solution comprising a prostaglandin derivative which is liable to be adsorbed to a container made of resin and hardly soluble in water as an active ingredient, wherein the prostaglandin derivative is selected in the group consisting of 16-phenoxy-15-deoxy-15, 15-difluoro-17,18,19,20-tetranorprostaglandin F2α; 16-(3-chlorophenoxy)-15-deoxy-15, 15-difluoro-17, 18, 19, 20-tetranorprostaglandin F2α; 16-phenoxy-15-deoxy-15,15-difluoro-13, 14-dihydro-17, 18, 19, 20-tetranorprostaglandin F2α;, alkyl esters thereof or salts thereof and the concentration of said prostaglandin derivative in the ophthalmic solution is prevented from lowering by adding a polysorbate 80 as nonionic surfactant and ethylenediaminetetraacetic acid or salts thereof as an antioxidant.

2. The ophthalmic solution as claimed in claim 1, wherein a concentration of said nonionic surfactant is at least five times that of said prostaglandin derivative.

3. A method of preventing a concentration lowering of a prostaglandin derivative which is liable to be adsorbed to a resinous container and hardly soluble in water, the prostaglandin derivative being contained in an ophthalmic solution as an active ingredient, wherein the prostaglandin derivative is selected in the group consisting of 16-phenoxy-15-deoxy-15, 15-difluoro-17,18,19,20-tetranorprostaglandin F2α; 16-(3-chlorophenoxy)-15-deoxy-15, 15-difluoro-17, 18, 19, 20-tetranorprostaglandin F2α; 16-phenoxy-15-deoxy-15,15-difluoro-13, 14-dihydro-17, 18, 19, 20-tetranorprostaglandin F2α; alkyl esters thereof or salts thereof and the concentration of said prostaglandin derivative in the ophthalmic solution is prevented from lowering by adding a polysorbate 80 as nonionic surfactant and as an antioxidant ethylenediaminetetraacetic acid or salts thereof as an antioxidant to the ophthalmic solution.

4. A method of inhibiting adsorption of a prostaglandin derivative which is liable to be adsorbed to a resinous container and hardly soluble in water, to the container which contains an ophthalmic solution comprising said prostaglandin derivative as an active ingredient, wherein the prostaglandin derivative is selected in the group consisting of 16-phenoxy-15-deoxy-15, 15-difluoro-17,18,19,20-tetranorprostaglandin F2α; 16-(3-chlorophenoxy)-15-deoxy-15, 15-difluoro-17, 18, 19, 20-tetranorprostaglandin F2α; 16-phenoxy-15-deoxy-15,15-difluoro-13, 14-dihydro-17, 18, 19, 20-tetranorprostaglandin F2α;, alkyl esters thereof or salts thereof and the concentration of said prostaglandin derivative in the ophthalmic solution is prevented from lowering by adding a polysorbate 80 as nonionic surfactant to the ophthalmic solution.

5. A method of inhibiting decomposition of a prostaglandin derivative which is liable to be adsorbed to a resinous container and hardly soluble in water, the prostaglandin derivative being contained in an ophthalmic solution as an active ingredient, wherein the prostaglandin derivative is selected in the group consisting of 16-phenoxy-15-deoxy-15, 15-difluoro-17,18,19,20-tetranorprostaglandin F2α; 16-(3-chlorophenoxy)-15-deoxy-15, 15-difluoro-17, 18, 19, 20-tetranorprostaglandin F2α; 16-phenoxy-15-deoxy-15,15-difluoro-13, 14-dihydro-17, 18, 19, 20-tetranorprostaglandin F2α;, alkyl esters thereof or salts thereof and the concentration of said prostaglandin derivative in the ophthalmic solution is prevented from lowering by adding ethylenediamine tetraacetic acid or salts thereof as antioxidant to the ophthalmic solution.

## Patentansprüche

1. Ophthalmische Lösung, die ein Prostaglandin-Derivat, das an einen Behälter aus Harz adsorbiert werden kann und in Wasser schwerlöslich ist, als Wirkstoff umfasst, wobei das Prostaglandin-Derivat aus der Gruppe ausgewählt ist, die aus 16-Phenoxy-15-desoxy-15,15-difluor-17,18,19,20-tetranorprostaglandin F2α, 16-(3-Chlorphenoxy)-15-desoxy-15,15-difluor-17,18,19,20-tetranorprostaglandin F2α, 16-Phenoxy-15-desoxy-15,15-difluor-13,14-dihydro-17,18,19,20-tetranorprostaglandin F2α, Alkylestern davon oder Salzen davon besteht, und das Absinken der Konzentration des Prostaglandin-Derivats in der ophthalmischen Lösung verhindert wird, indem man Polysorbat 80 als nichtionisches Tensid und Ethylendiamintetraessigsäure oder Salze davon als Antioxidans hinzufügt.

2. Ophthalmische Lösung gemäß Anspruch 1, wobei die Konzentration des nichtionischen Tensids wenigstens fünfmal so groß ist wie die des Prostaglandin-Derivats.

3. Verfahren zur Verhinderung des Absinkens der Konzentration eines Prostaglandin-Derivats, das an einen Harzbehälter adsorbiert werden kann und in Wasser schwerlöslich ist, wobei das Prostaglandin-Derivat in einer ophthalmischen Lösung als Wirkstoff enthalten ist, wobei das Prostaglandin-Derivat aus der Gruppe ausgewählt ist, die aus 16-Phenoxy-15-desoxy-15,15-difluor-17,18,19,20-tetranorprostaglandin F2α, 16-(3-Chlorphenoxy)-15-desoxy-15,15-difluor-17,18,19,20-tetranorprostaglandin F2α, 16-Phenoxy-15-desoxy-15,15-difluor-13,14-dihydro-17,18,19,20-tetranorprostaglandin F2α, Alkylestern davon oder Salzen davon besteht, und das Absinken der Konzentration des Prostaglandin-Derivats in der ophthalmischen Lösung verhindert wird, indem man Polysorbat 80 als nichtionisches Tensid und Ethylendiamintetraessigsäure oder Salze davon als Antioxidans zu der ophthalmischen Lösung gibt.

4. Verfahren zum Hemmen der Adsorption eines Prostaglandin-Derivats, das an einen Harzbehälter adsorbiert werden kann und in Wasser schwerlöslich ist, an dem Behälter, der eine ophthalmische Lösung enthält, die das Prostaglandin-Derivat als Wirkstoff enthält, wobei das Prostaglandin-Derivat aus der Gruppe ausgewählt ist, die aus 16-Phenoxy-15-desoxy-15,15-difluor-17,18,19,20-tetranorprostaglandin F2α, 16-(3-Chlorphenoxy)-15-desoxy-15,15-difluor-17,18,19,20-tetranorprostaglandin F2α, 16-Phenoxy-15-desoxy-15,15-difluor-13,14-dihydro-17,18,19,20-tetranorprostaglandin F2α, Alkylestern davon oder Salzen davon besteht, und das Absinken der Konzentration des Prostaglandin-Derivats in der ophthalmischen Lösung verhindert wird, indem man Polysorbat 80 als nichtionisches Tensid zu der ophthalmischen Lösung gibt.

5. Verfahren zum Hemmen der Zersetzung eines Prostaglandin-Derivats, das an einen Harzbehälter adsorbiert werden kann und in Wasser schwerlöslich ist, wobei das Prostaglandin-Derivat in einer ophthalmischen Lösung als Wirkstoff enthalten ist, wobei das Prostaglandin-Derivat aus der Gruppe ausgewählt ist, die aus 16-Phenoxy-15-desoxy-15,15-difluor-17,18,19,20-tetranorprostaglandin F2α, 16-(3-Chlorphenoxy)-15-desoxy-15,15-difluor-17,18,19,20-tetranorprostaglandin F2α, 16-Phenoxy-15-desoxy-15,15-difluor-13,14-dihydro-17,18,19,20-tetranorprostaglandin F2α, Alkylestern davon oder Salzen davon besteht, und das Absinken der Konzentration des Prostaglandin-Derivats in der ophthalmischen Lösung verhindert wird, indem man Ethylendiamintetraessigsäure oder Salze davon als Antioxidans zu der ophthalmischen Lösung gibt.

## Revendications

1. Solution ophtalmique comprenant un dérivé de prostaglandine qui est susceptible d'être adsorbé sur un récipient fait de résine et difficilement soluble dans l'eau en tant que principe actif, dans laquelle le dérivé de prostaglandine est choisi dans le groupe comprenant la 16-phénoxy-15-désoxy-15,15-difluoro-17,18,19,20-tétranorprostaglandine F2α ; la 16-(3-chlorophénoxy)-15-désoxy-15,15-difluoro-17,18,19,20-tétranorprostaglandine F2α ; la 16-phénoxy-15-désoxy-15,15-difluoro-13,14-dihydro-17,18,19,20-tétranorprostaglandine F2α ; les esters alkyliques de celles-ci ou les sels de celles-ci et la baisse de la concentration dudit dérivé de prostaglandine dans la solution ophtalmique est empêchée par l'addition d'un polysorbate 80 en tant que tensioactif non ionique et d'acide éthylènediaminetétraacétique ou de sels de celui-ci en tant qu'antioxydant.

2. Solution ophtalmique selon la revendication 1, dans laquelle une concentration dudit tensioactif non ionique est égale à au moins cinq fois celle dudit dérivé de prostaglandine.

3. Procédé destiné à empêcher la baisse d'une concentration d'un dérivé de prostaglandine qui est susceptible d'être adsorbé sur un récipient résineux et difficilement soluble dans l'eau, le dérivé de prostaglandine étant contenu dans une solution ophtalmique en tant que principe actif, dans lequel le dérivé de prostaglandine est choisi dans le groupe comprenant la 16-phénoxy-15-désoxy-15,15-difluoro-17,18,19,20-tétranorprostaglandine F2α ; la 16-(3-chlorophénoxy)-15-désoxy-15,15-difluoro-17,18,19,20-tétranorprostaglandine F2α ; la 16-phénoxy-15-désoxy-15,15-difluoro-13,14-dihydro-17,18,19,20-tétranorprostaglandine F2α ; les esters alkyliques de celles-ci ou les sels de celles-ci et la baisse de la concentration dudit dérivé de prostaglandine dans la solution ophtalmique est empêchée par l'addition, à la solution ophtalmique, d'un polysorbate 80 en tant que tensioactif non ionique et d'acide éthylènediaminetétraacétique ou de sels de celui-ci en tant qu'antioxydant.

4. Procédé destiné à inhiber l'adsorption d'un dérivé de prostaglandine qui est susceptible d'être adsorbé sur un récipient résineux et difficilement soluble dans l'eau, sur le récipient qui contient une solution ophtalmique comprenant ledit dérivé de prostaglandine en tant que principe actif, dans lequel le dérivé de prostaglandine est choisi dans le groupe comprenant la 16-phénoxy-15-désoxy-15,15-difluoro-17,18,19,20-tétranorprostaglandine F2α ; la 16-(3-chlorophénoxy)-15-désoxy-15,15-difluoro-17,18,19,20-tétranorprostaglandine F2α ; la 16-phénoxy-15-désoxy-15,15-difluoro-13,14-dihydro-17,18,19,20-tétranorprostaglandine F2α ; les esters alkyliques de celles-ci ou les sels de celles-ci et la baisse de la concentration dudit dérivé de prostaglandine dans la solution ophtalmique est empêchée par l'addition, à la solution ophtalmique, d'un polysorbate 80 en tant que tensioactif non ionique.

5. Procédé destiné à inhiber la décomposition d'un dérivé de prostaglandine qui est susceptible d'être adsorbé sur un récipient résineux et difficilement soluble dans l'eau, le dérivé de prostaglandine étant contenu dans une solution ophtalmique en tant que principe actif, dans lequel le dérivé de prostaglandine est choisi dans le groupe comprenant la 16-phénoxy-15-désoxy-15,15-difluoro-17,18,19,20-tétranorprostaglandine F2α ; la 16-(3-chlorophénoxy)-15-désoxy-15,15-difluoro-17,18,19,20-tétranorprostaglandine F2α ; la 16-phénoxy-15-désoxy-15,15-difluoro-13,14-dihydro-17,18,19,20-tétranorprostaglandine F2α ; les esters alkyliques de celles-ci ou les sels de celles-ci et la baisse de la concentration dudit dérivé de prostaglandine dans la solution ophtalmique est empêchée par l'addition, à la solution ophtalmique, d'acide éthylènediaminetétraacétique ou de sels de celui-ci en tant qu'antioxydant.
